# FASCICULE DE BREVET EUROPEEN

(11) **EP 1 575 605 B1**
(45) Date de publication et mention de la délivrance du brevet: **13.04.2011**
(21) Numéro de dépôt: 03767892.7
(22) Date de dépôt: 04.11.2003
(51) Int. Cl.: A61K 38/06, A61K 38/07, A61K 38/08, A61Q 17/00, A61Q 19/00, A61P 17/00

(54) **COMPOSITION COSMETIQUE OU PHARMACEUTIQUE COMPRENANT DES PEPTIDES POSSEDANT LE MOTIF ARG-GLY-SER**
PHARMAZEUTISCHE, KOSMETISCHE ODER THERAPEUTISCHE ZUSAMMENSETZUNGEN ENTHALTEND PEPTIDE MIT DEM MOTIV ARG-GLY-SER
COSMETIC OR PHARMACEUTICAL COMPOSITION COMPRISING PEPTIDES WITH THE SEQUENCE ARG-GLY-SER

(30) Priorité: 08.11.2002 FR 0214012; 13.08.2003 FR 0309889
(43) Date de publication de la demande: 21.09.2005
(73) Titulaire: Société d'Extraction des Principes Actifs (Vincience SA), 06410 Biot (FR)
(72) Inventeur: DAL FARRA, Claude, F-06650 Opio (FR); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2003/003280
(87) Numéro de publication internationale: WO 2004/043482

(56) Documents cités:
- EP-A- 0 764 444
- WO-A2-03/064614
- FR-A- 2 788 777
- US-B1- 6 228 989
- DUDA TERESA ET AL: "Core sequence of ATP regulatory module in receptor guanylate cyclases." FEBS (FEDERATION OF EUROPEAN BIOCHEMICAL SOCIETIES) LETTERS, vol. 315, no. 2, 1993, pages 143-148, XP002243861 ISSN: 0014-5793

## Description

L'invention concerne le domaine de la cosmétique et de la pharmaceutique, notamment le domaine de la dermatologie.

La présente invention a pour objet une composition cosmétique et/ou dermatologique et/ou pharmaceutique comprenant comme principe actif au moins un peptide de séquence (AA)ₙ-Arg-Gly-Ser-(AA)ₙ, dans laquelle (AA) est un quelconque acide aminé ou un de ses dérivés, n étant compris entre 0 et 3.

La peau est un organe de revêtement recouvrant la totalité de la surface du corps. C'est un organe vital assurant des fonctions multiples telles que des fonctions sensitives, protectrices vis à vis d'agressions externes multiples, immunitaires, métaboliques ou encore thermorégulatrices. Ces rôles sont rendus possibles grâce à une structure complexe qui associe des structures tissulaires variées. La peau est constituée de trois compartiments distincts superposés : l'épiderme, le derme et l'hypoderme. L'épiderme est un épithélium de revêtement qui constitue la structure externe de la peau et assure sa fonction de protection. Cette fonction est assurée par la cohésion des cellules épithéliales et par la production d'une protéine filamenteuse et résistante, la kératine. Le derme est un tissu conjonctif constitué d'une substance fondamentale dans laquelle baignent les fibroblastes, des fibres de collagène et des fibres d'élastine, fibres protéiques synthétisées par les fibroblastes. Les fibres de collagène assurent une grande partie de la solidité du derme, elles participent à l'élasticité et surtout à la tonicité de la peau et/ou des muqueuses. Au-dessous du derme se trouve une couche de tissus adipeux : l'hypoderme. L'hypoderme est constitué d'une couche de graisse de réserve, ou tissus adipeux blanc, rattaché à la partie inférieure du derme par des expansions de fibres de collagènes et de fibres élastiques. Il est constitué de grosses cellules vacuolisées, les adipocytes, presque entièrement remplies de triglycérides. Ces cellules peuvent changer rapidement de volume, lors d'un amaigrissement ou d'une prise de poids, et peuvent mesurer de 40 à 120 µm de diamètre, ce qui correspond à une variation de 27 fois en volume. Le tissu adipeux contient également du tissu conjonctif dans lequel se trouvent, entre autres, des fibroblastes particuliers et des préadipocytes. Ce tissu adipeux est capable de stoker des lipides sous forme de triglycérides ou de les libérer sous forme d'acides gras et de glycérol.

Aujourd'hui, les professionnels de la santé et de la cosmétique mettent en oeuvre de plus en plus de moyens afin de découvrir de nouveaux principes actifs capables d'agir sur la peau. Cette industrie recherche des actifs non seulement capables de la protéger et de l'entretenir mais aussi des actifs capables d'améliorer son aspect ainsi que le bien-être des individus qui l'utilisent. On exige de ces nouveaux produits qu'ils possèdent simultanément plusieurs propriétés et qu'ils présentent, par conséquent, un spectre de performances amélioré. Ces actifs se doivent donc d'avoir un mode d'action général sur la peau, et ont donc à agir à différents niveaux.

Le but de la présente invention est de mettre à disposition une nouvelle substance, utilisable dans le domaine de la cosmétique et de la pharmacie, qui présente, outre des propriétés de soins de la peau, une action préventive et curative sur les manifestations du vieillissement cutané, mais aussi une action stimulante et revitalisante. Cet actif pourra donc, à la fois, combattre les phénomènes du vieillissement cutané, protéger la peau d'une manière efficace, ainsi que, par exemple, avoir une action amincissante.

L'Adénosine 5'-triphosphate (ATP) est une molécule qui joue un rôle central dans beaucoup de mécanismes cellulaires. C'est, en effet, la source d'énergie principale des cellules : cette molécule est capable de mettre en réserve de l'énergie chimique et de la restituer très facilement dans les réactions qui nécessitent de l'énergie pour se produire. L'ATP joue un rôle particulièrement important au niveau de la peau : c'est un marqueur de la vitalité et de l'activité des cellules. Puisque, en effet, l'activité cellulaire corrèle étroitement avec, par exemple, une augmentation de la synthèse de molécules essentielles telles que les protéines ou l'ADN.

De ce fait, en augmentant la quantité d'ATP intracellulaire, on stimule la cellule et on lui apporte l'énergie nécessaire afin de synthétiser les enzymes qui induiront des mécanismes d'activation et qui permettront ainsi d'augmenter le métabolisme cellulaire. Ainsi, en stimulant par exemple la synthèse des molécules essentielles au bon fonctionnement de la peau, telles que les protéines de la matrice extracellulaire (le collagène, l'élastine, la fibronectine) ou bien la kératine, on permettra alors à la peau de mieux lutter contre les phénomènes du vieillissement et de favoriser son renouvellement (par une augmentation de la prolifération et de la différentiation cellulaire). Elle pourra aussi mieux développer son processus de réparation ou, encore, lutter plus efficacement contre les dommages occasionnés par les UV.

Les inventeurs ont réussi à sélectionner des substances particulières présentant des propriétés remarquables lorsque celles-ci sont appliquées sur la peau.

De manière inattendue, les inventeurs ont découvert que les peptides correspondant à la formule générale (T) :

(AA)n-Arg-Gly-Ser-(AA)n (I)

dans laquelle (AA) est un acide aminé quelconque, ou un de ses dérivés, et n est un entier compris entre 0 et 3, ont des propriétés remarquables en tant qu'agent de soin pour la peau.

L'actif ainsi obtenu possède des effets particulièrement remarquables au niveau de la peau. Outre ses propriétés de soin, il possède une véritable action stimulante et revitalisante sur la peau et sur les cellules qui la compose. Ce composé possède ainsi des propriétés amincissantes et anti-cellulite, des propriétés protectrices, mais aussi une action très efficace dans la lutte contre les manifestations du vieillissement cutané. En effet, il a été découvert que ce peptide a un effet sur la modulation de la concentration d'ATP dans la cellule, sur la concentration intracellulaire de calcium ainsi que sur la production et l'activation de protéines essentielles à la peau.

**Selon un premier aspect, la présente invention a pour objet** une composition cosmétique et/ou dermatologique et/ou pharmaceutique, caractérisée en ce qu'elle contient, dans un milieu acceptable, comme principe actif, au moins un peptide de formule (I):

(AA)n-Arg-Gly-Ser-(AA)n (I)

dans laquelle (AA) est un quelconque acide aminé ou un de ses dérivés, n est un entier compris entre 0 et 3, le peptide étant présent dans la composition à une concentration comprise entre 0,005 et 500 ppm environ.

Le terme "acide aminé" se réfère ici à tout acide organique naturel ou non naturel ayant la formule (II) :

-NHR-CR-C(O)-O- (II)

où chaque -R est indépendamment sélectionné entre un hydrogène et un groupement alkyl ayant entre 1 et 12 atomes de carbone. Préférentiellement, au moins un groupement -R de chaque acide aminé est un hydrogène.

Par le terme "alkyl", on entend ici une chaîne carbonée pouvant être linéaire ou ramifiée, substituée (mono- ou poly-) ou non-substituée ; saturée, mono-saturée (une double ou triple liaisons dans la chaîne) ou poly-insaturées (deux ou plusieurs doubles liaisons, deux ou plusieurs triples liaisons, une ou plusieurs doubles liaisons et une ou plusieurs triples liaisons dans la chaîne).

A la connaissance de la demanderesse, il n'a jamais été décrit dans l'art antérieur l'utilisation d'un peptide de séquence (AA)n-Arg-Gly-Ser-(AA)n dans laquelle (AA) est un acide aminé quelconque, ou un de ses dérivés, et n un entier compris entre 0 et 3, le peptide étant présent dans la composition à une concentration comprise entre 0,005 et 500 ppm environ, en cosmétique et/ou en dermatologie et/ou en pharmaceutique.

Le peptide utilisé selon l'invention peut contenir notamment de 3 à 9 résidus d'acides aminés, et en particulier 3, 4 ou 5 résidus d'acides aminés.

L'invention concerne notamment l'utilisation de peptides contenant au moins la séquence peptidique Arginine-Glycine-Serine ainsi que l'utilisation de dérivés de ces peptides. Selon un mode de réalisation actuellement préféré, le peptide précité est préférentiellement le peptide de séquence Arg-Gly-Ser.

Lorsque l'on utilise un peptide contenant le tripeptide Arginine-Glycine-Serine, il est bien entendu que celui-ci est choisi de telle sorte que les acides aminés entourant le motif Arginine-Glycine-Serine, tant par leur nature que par la structure secondaire du peptide qu'ils vont induire, n'empêchent pas celui-ci d'exercer l'activité pour laquelle il est utilisé dans la présente invention.

Les dérivés d'acides aminés et les dérivés de peptides sont, par exemple, ceux dont au moins un groupement fonctionnel (en particulier les groupements amines et carboxyliques) est protégé avec un groupement protecteur. En effet, il se peut que pour des questions de résistance à la dégradation, il soit nécessaire d'utiliser selon l'invention une forme protégée du peptide. La forme de protection doit évidemnent être une forme biologiquement compatible et doit être compatible avec une utilisation dans le domaine des cosmétiques ou de la pharmacie.

De nombreuses formes de protection biologiquement compatibles peuvent être envisagées, elles sont bien connues de l'homme du métier, comme par exemple l'acylation ou l'acétylation de l'extrémité amino-terminale, ou l'amidation ou l'estérification de l'extrémité carboxy-terminale. Ainsi, l'invention concerne une utilisation telle que définie précédemment caractérisée par le fait que le peptide est sous forme protégée ou non. De préférence, on utilise une protection basée soit sur l'acylation ou l'acétylation de l'extrémité amino-terminale, soit sur l'amidation ou l'estérification de l'extrémité carboxy-terminale, soit encore des deux.

Les dérivés d'acides aminés et les dérivés de peptides concernent aussi les acides aminés et les peptides reliés entre eux par une liaison pseudo-peptidique. On entend par "liaison pseudo-peptidique" tous les types de liaisons susceptibles de remplacer les liaisons peptidiques "classiques".

Dans le domaine des acides aminés, la géométrie des molécules est telle qu'elles peuvent théoriquement se présenter sous la forme d'isomères optiques différents. Il existe en effet une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un peptide d'origine naturelle ne sera constitué que d'acides aminés de type L-aa. Cependant la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre ou lévogyre. Ainsi, les acides aminés constituant le peptide selon l'invention, peuvent être sous configuration L- et D-, de manière préférentielle, les acides aminés sont sous forme L. Le peptide selon l'invention peut donc être sous forme L-, D- ou DL-.

Les peptides, objets du présent brevet, peuvent être obtenus soit par synthèse chimique classique (en phase solide ou en phase homogène liquide), soit par synthèse enzymatique (Kullman et al., J. Biol. Chem. 1980, 225, 8234) à partir d'acides aminés constitutifs ou de leurs dérivés. Les peptides selon l'invention peuvent être aussi obtenus par fermentation d'une souche de bactéries modifiées ou non, par génie génétique pour produire les peptides de séquence, indiquée précédemment, et leurs fragments, ou encore par extraction de protéines d'origine animale ou végétale, préférentiellement d'origine végétale, suivie d'une hydrolyse contrôlée qui libère les fragments peptidiques de tailles moyennes et de petites tailles dont il est question, avec la condition que les éléments libérés doivent contenir au moins la séquence Arg-Gly-Ser.

De très nombreuses protéines trouvées dans les plantes sont susceptibles de contenir ces séquences au sein de leur structure. L'hydrolyse ménagée permet de dégager ces fragments peptidiques. Il est possible, mais non nécessaire pour réaliser l'invention, d'extraire soit les protéines concernées d'abord et de l'hydrolyser ensuite, soit d'effectuer l'hydrolyse d'abord sur un extrait brut et de purifier les fragments peptidiques ensuite. D'autres procédés plus simples ou plus complexes peuvent être envisagés par l'homme du métier connaissant le métier de synthèse, d'extraction et de purification des protéines et des peptides. Ainsi le peptide selon l'invention peut être un peptide d'origine naturelle ou synthétique. Préférentiellement selon l'invention, le peptide est obtenu par synthèse chimique.

Selon un mode de réalisation avantageux de l'invention, le peptide précité cst préalablement solubilisé dans un ou plusieurs solvants cosmétiquement ou pharmaceutiquement acceptable, classiquement utilisé par l'homme du métier, comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, les peptides précités sont préalablement solubilisés dans un vecteur cosmétique ou pharmaceutique comme les liposomes ou adsorbés sur des polymères organiques poudreux, des supports minéraux comme les talcs et bentonites, et plus généralement solubilisés dans, uu fixés sur, tout vecteur cosmétiquement ou pharmaceutiquement acceptable.

Il est bien entendu évident que le peptide selon l'invention peut être utilisé seul ou bien en association avec au moins un autre agent actif, dans ou pour la préparation d'une composition cosmétique et/ou dermatologique et/ou pharmaceutique.

Selon un autres aspect, l'invention a également pour objet un procédé de traitement cosmétique non thérapeutique consistant à appliquer une quantité efficace d'une composition selon l'invention, pour le soin et le traitement de la peau et/ou des phanères permettant d'activer le métabolisme énergétique cellulaire ; un procédé de traitement cosmétique non thérapeutique consistant à appliquer une quantité efficace d'une composition selon l'invention, pour le soin et le traitement de la peau et/ou des phanères présentant des propriétés cytostimulantes, notamment en tant qu'agent de soin favorisant la régénération tissulaire ; un procédé de traitement cosmétique non thérapeutique consistant à appliquer une quantité efficace d'une composition selon l'invention, pour stimuler la synthèse des protéines de la matrice extracellulaire et/ou stimuler la synthèse de kératines ; un procédé de traitement cosmétique non thérapeutique consistant à appliquer une quantité efficace d'une composition selon l'invention, pour lutter et/ou prévenir contre les manifestations du vieillissement cutané ; un procédé de traitement cosmétique non thérapeutique consistant à appliquer une quantité efficace d'une composition selon l'invention, pour protéger la peau et/ou les cheveux contre tous types d'agressions extérieures ; et un procédé de traitement cosmétique non thérapeutique consistant à appliquer une quantité efficace d'une composition selon l'invention, contre la cellulite et/ou la peau d'orange; et/ou afin de réduire, éliminer ou prévenir les surcharges graisseuses sous-cutanées.

Le peptide selon l'invention est avantageusement utilisé comme agent de soin et de traitement pour la peau et/ou les phanères. On entend par agent de soin et de traitement, au sens de la présente invention, des agents qui présentant, de façon générale, une activité réparatrice et revitalisante permettant, entre autres, à la peau et/ou aux phanères de mieux réagir aux agressions qu'elles peuvent rencontrer.

Ce peptide possède une action favorable au niveau du métabolisme énergétique des cellules de la peau, telles que les fibroblastes et les adipocytes, ainsi qu'une activité cytostimulante. Le peptide selon l'invention est avantageusement utilisé en tant qu'agent de soin et de traitement pour la peau et/ou les phanères permettant d'activer le métabolisme énergétique cellulaire. Par agent permettant d'activer le métabolisme énergétique cellulaire, on entend, par exemple, des composés capables d'augmenter la synthèse d'ATP intracellulaire des cellules de la peau ou encore capables d'augmenter la concentration de calcium intracellulaire. En effet, il a été démontré que le peptide selon l'invention permet

d'augmenter la synthèse d'ATP intracellulaire, notamment, au niveau des fibroblastes et des adipocytes ; mais aussi qu'il est capable de provoquer une hausse de la concentration du calcium intracellulaire des cellules de la peau. Cette stimulation permet d'activer différents mécanismes favorables à la cellule, notamment afin de l'aider à lutter contre le stress et le vieillissement. Le peptide selon l'invention peut aussi avoir une action antioxydante.

L'activité cytostimulante et revitalisante du peptide selon l'invention se caractérise par une augmentation de la différentiation et de la régénération cellulaire. En effet, le peptide selon l'invention favorise la différentiation cellulaire, notamment celle des kératinocytes. Ces derniers migrent plus rapidement vers la surface de l'épiderme et assurent une meilleure résistance de la couche cornée. En progressant vers les couches supérieures, les kératinocytes s'aplatissent et déversent dans l'espace extracellulaire un ciment constitué de lipides, cholestérol, acides gras libres saturés et céramides qui augmentent la cohésion entre les cellules et contribuent ainsi au rôle de barrière de l'épiderme. Le peptide augmente donc de manière efficace la fonction de barrière cutanée de la peau et favorise ainsi la régénération tissulaire.

Le composé selon la présente invention, par son action cytostimulante, va intervenir de manière efficace au niveau de tous les phénomènes liés au vieillissement cutané et au de stress de la cellule. La réserve énergétique constituée par le peptide selon l'invention améliore la synthèse des protéines au niveau des cellules de la peau et/ou améliore leur stabilité, tout particulièrement au niveau des cellules épidermiques.

Il a été démontré que les peptides, selon l'invention, ont des effets bénéfiques sur les protéines de la matrice extracellulaire, notamment qu'ils permettent d'augmenter et de favoriser leur synthèse. Par protéine de la matrice extracellulaire, on entend, par exemple, des protéines telles que le collagène, la fibronectine ou encore l'élastine. De la même manière, il a été démontré que le peptide de formule (I) possède une action effice au niveau des kératinocytes. L'invention a donc pour autre objet l'utilisation d'au moins un peptide tel que défini précédemment, dans ou pour la préparation d'une composition, afin de stimuler la synthèse de kératines.

Le peptide selon l'invention est ainsi particulièrement bien adapté à une utilisation afin de lutter et/ou prévenir contre les phénomènes du vieillissement cutané. Par modifications cutanées du vieillissement, on entend toutes modifications de l'aspect extérieur de la peau dues au vieillissement, comme, par exemple, les rides et ridules, la peau flétrie, la peau molle, la peau amincie, le manque d'élasticité et/ou de tonus de la peau, la peau terne et sans éclat mais également toutes modifications internes de la peau qui ne se traduisent pas systématiquement par un aspect extérieur modifié comme, par exemple, toutes dégradations internes de la peau consécutives à une exposition aux rayonnements ultra-violets.

L'augmentation de la concentration d'ATP va aussi permettre à la cellule d'être protégée et de mieux résister au stress que produit sur elle l'environnement. La cellule va donc être protégée contre tous types d'agressions extérieures. La présente invention concerne donc l'utilisation d'au moins un peptide de formule (I) tel que défini précédemment, dans ou pour la préparation d'une composition, afin de protéger la peau et/ou les cheveux contre tous types d'agressions extérieures. On entend par le terme "agression extérieure" les agressions que peut produire l'environnement. Ces agressions peuvent être d'origine chimique, physique, biologique ou thermique. A titre d'exemple, on peut citer des agressions telles que la pollution, les UV, les frottements, l'eau à forte concentration de calcaire, les variations de température ou encore les produits à caractère irritant tels que les tensioactifs, les conservateurs ou les parfums.

Par ailleurs, par son activité stimulante, le peptide possède une activité particulière et prononcée sur la lipolyse, ce qui le rend particulièrement utile dans des préparations à visée amincissante. Il a, en effet, été constaté que le peptide selon l'invention, ou la composition le contenant, possède une action efficace au niveau des adipocytes. Ce peptide peut être ainsi utilisé dans ou pour la fabrication d'une composition cosmétique et/ou pharmaceutique, à usage topique, destinée au traitement de la cellulite et/ou au traitement de la peau d'orange. Il est utilisé d'une manière plus générale afin de réduire, éliminer ou prévenir les surcharges graisseuses sous-cutanées. La cellulite est une configuration particulière du tissu adipeux. Elle désigne un aspect matelassé et capitonné de la peau qui correspond, de façon schématique, à l'accentuation de tissus adipeux dans certaines régions du corps due à une augmentation de la quantité de graisse, stockée dans les adipocytes, dont le volume et le nombre augmente. A un stade avancé de la formation de cellulite, la peau prend spontanément l'aspect de « peau d'orange ».

Le peptide selon l'invention contribue à faire diminuer significativement la quantité de triglycérides contenue dans les vacuoles adipocytaires. Ce phénomène est dû à une augmentation du phénomène de lipolyse dans les adipocytes, mécanisme qui passe par une augmentation de la quantité d'ATP intracellulaire suivie d'une augmentation de la quantité d'AMPc intracellulaire. Cette augmentation de la quantité d'AMPc a pour conséquence une stimulation accrue de l'activité de la Triglycéride-lipase, enzyme qui permet l'hydrolyse des triglycérides en acides gras. La lipolyse, étant la réaction d'élimination des triglycérides stockés dans les adipocytes, une augmentation de ce phénomène permettra une élimination plus importante des triglycérides ainsi qu'une augmentation du relargagé des acides gras libres et du glycérol dans le milieu extracellulaire. Ainsi, lorsque la quantité de triglycérides présents dans les vacuoles adipocytaires diminue, leur volume décroît. La peau reprend ainsi petit à petit son aspect « normal » : le tissu cellulitique est diminué, l'effet peau d'orange est atténué, l'aspect disgracieux du corps s'estompe peu à peu. Ainsi, le degré d'activité lipolytique de peptide selon l'invention et sa capacité à agir sur la lipolyse, va varier en fonction de la quantité d'AMPc. Le peptide va donc permettre de diminuer, ralentir ou résorber les dépôts graisseux. L'actif selon l'invention ou la composition le contenant aura une activité amincissante et permettra d'améliorer l'aspect de la peau et, en particulier, d'atténuer l'aspect « peau d'orange ».

D'une façon plus générale, la composition selon l'invention contient le composé actif tel que défini précédemment. Ainsi, le peptide présent dans la composition est choisi parmi les peptides dont au moins un groupement fonctionnel est protégé par un groupement protecteur, ce groupement protecteur étant soit une acylation ou une acétylation de l'extrémité amino-terminale, soit sur une amidation ou une estérification de l'extrémité carboxy-terminale, soit les deux.

Il est bien entendu que le peptide selon l'invention peut être utilisé seul ou en association avec au moins un autre agent actif,

Dans la composition selon l'invention, le peptide peut être un mélange de dérivés peptidiqucs et/ou constitué de dérivés d'acides aminés.

La composition contenant le peptide selon l'invention peut être une composition cosmétique ou dermatologique ou pharmaceutique. Préférentiellement selon l'invention, la composition est une composition cosmétique, car elle est destinée à améliorer l'aspect et les performances cutanées générales de l'individu qui en fait usage. La composition selon l'invention est préférentiellement une composition cosmétique et/ou dermatologique adaptée à l'administration par voie topique cutanée comprenant un milieu cosmétiquement ou pharmaceutiquement acceptable.

Il est bien évident que l'invention s'adresse aux mammifères en général et plus particulièrement aux êtres humains.

La quantité efficace de principe actif correspond à la quantité nécessaire pour obtenir le résultat désiré. Selon un mode de réalisation avantageux de l'invention, le peptide précité est présent dans les compositions de l'invention à une concentration comprise entre 0,005 et 500 ppm (parties par million) environ, et préférentiellement à une concentration comprise entre 0,1 et 50 ppm environ par rapport au poids total de la composition finale.

Quelle que soit la forme de l'invention, la composition selon l'invention peut être ingérée, injectée ou appliquée sur la peau (sur toute zone cutanée du corps), les cheveux, les ongles ou les muqueuses. Selon le mode d'administration, la composition selon l'invention peut se présenter sous toutes les formes galéniques normalement utilisées. Préférentiellement, les compositions selon la présente invention se présenteront sous une forme galénique adaptée à l'administration par voie topique cutanée, et couvrent toutes les formes cosmétiques ou dermatologiques. Ces compositions doivent donc contenir un milieu cosmétiquement acceptable, c'est-à-dire compatible avec la peau, les muqueuses, les poils ou les cheveux. Ces compositions pourront notamment se présenter sous forme d'une solution aqueuse, hydralcoolique ou huileuse ; d'une émulsion huile-dans-eau, eau-dans-huile ou émulsions multiples ; elles aussi peuvent se présenter sous forme de crèmes, de suspensions, ou encore poudres, adaptées à une application sur la peau, les muqueuses, les lèvres et/ou les cheveux. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, en outre, tout additif usuellement utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc. Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme du métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Les compositions selon l'invention trouvent une application notamment comme compositions cosmétiques ou pharmaceutiques pour la peau, les muqueuses et/ou les semimuqueuses, mais aussi comme compositions cosmétiques ou pharmaceutiques pour les phanères et/ou les cheveux. Elles trouvent une application toute particulière en tant que produit de protection et/ou de soin de la peau, ou encore en tant que composition anti-ride et/ou anti-âge, ou encore en tant que composition amincissante et/ou raffermissante. La composition amincissante et/ou raffermissante pourra être appliquée localement sur les zones du visage ou du corps à affiner, en particulier sur les hanches, les fesses, les cuisses, le ventre, le visage. On peut également envisager une application dans le domaine des compositions de maquillage de la peau du visage et du corps, telles que les rouges à lèvres, les fond de teint, les crèmes teintées, les sticks anti-cernes, ou les compositions anti-solaires ou de bronzage artificiel.

Les compositions, objet de l'invention, trouvent leur application dans grand nombre de traitements notamment cosmétiques ou dermatologiques, et elles peuvent constituer une composition cosmétique, notamment pour le traitement, la protection, le soin, le démaquillage et/ou le nettoyage de la peau, des lèvres et/ou des cheveux, et/ou pour le maquillage de la peau, des lèvres, des cils et/ou du corps. La composition selon l'invention peut également consister en des préparations solides comprenant également des savons ou des pains de nettoyage. La composition peut être aussi conditionnée sous forme d'une composition pour aérosol comprenant également un agent propulseur sous pression. La composition peut être aussi à usage bucco-dentaire, par exemple une pâte de dentifrice La composition de l'invention peut aussi être une composition cosmétique destinée à une administration par voie orale. Pour une administration par voie orale, la composition selon l'invention peut se présenter sous toutes formes adaptées, particulièrement sous forme d'une solution buvable, d'un sirop, d'un comprimé, d'une dragée, d'une gélule, ou encore d'un aliment ou complément nutritionnel. Selon l'invention, on peut ajouter à la composition de l'invention d'autres agents actifs destinés, entre autres, à la prévention et/ou au traitement des manifestations cutanées du vieillissement, ou bien destiné à la protection de la peau contre les agressions extérieures, ou encore des actifs destinés au traitement de la cellulite et au phénomène de « peau d'orange ».

Selon un autre aspect, la présente invention concerne un procédé de traitement cosmétique non thérapeutique destiné à traiter les peaux âgées et/ou à combattre et/ou prévenir les phénomènes de vieillissement cutané consistant à appliquer, sur la surface de la peau, une quantité efficace d'une composition telle que définie précédemment. C'est-à-dire une composition contenant le peptide correspondant à la formule générale (1), afin d'obtenir l'action désirée. La présente invention concerne, de la même manière, un procédé de traitement cosmétique non thérapeutique afin de protéger la peau et/ou les cheveux contre tous types d'agressions extérieures. Selon un autre aspect de l'invention, la présente invention concerne un procédé de traitement cosmétique non thérapeutique afin de favoriser la différenciation cellulaire et/ou de favoriser la régénération tissulaire et/ou de renforcer la barrière cutanée de la peau consistant à appliquer, sur la surface de la peau, une quantité efficace d'une composition telle que définie précédemment. La présente invention concerne aussi un procédé de soin cosmétique non thérapeutique afin d'obtenir une action amincissante, ainsi qu'un procédé de soin cosmétique non thérapeutique destiné à réduire, éliminer et/ou prévenir les surcharges graisseuses sous-cutanées, et/ou destiné à lutter contre la cellulite et/ou à lutter contre le phénomène de peau d'orange, consistant à

appliquer, typiquement, sur les zones concernées de la peau, une quantité efficace d'une composition telle que définie précédemment, Des modes de réalisation particuliers de ce procédé de traitement cosmétique résultent également de la description précédente.

Le procédé de traitement cosmétique de l'invention peut être mis en oeuvre notamment en appliquant les compositions cosmétiques telles que définies ci-dessus, selon la technique d'utilisation habituelle de ces compositions, par exemple : application de crèmes, de gels, de sérums, de lotions, de laits, de shampooings ou de compositions anti-solaires, sur la peau ou sur les cheveux, ou encore, application de dentifrice sur les gencives.

D'autres avantages et caractéristiques de l'invention apparaîtront mieux à la lecture des exemples donnés à titre illustratif et non limitatif.

### Exemple 1 - Mise en évidence de l'effet du peptide sur la quantité d'ATP intracellulaire.

Le but de cette étude est de déterminer l'influence du peptide selon l'invention sur la production d'ATP par différentes cellules de la peau. Cette étude s'effectue à l'aide d'un Kit, "ATP Bioluminescence Assay Kit HS II", qui permet d'apprécier le taux d'ATP intracellulaire. L'étude est effectuée sur des fibroblastes ainsi que sur une lignée cellulaire préadipocytaire 3T3-L1 différenciée.

Le test a été effectué, pour une part, sur des fibroblastes. Ces fibroblastes ont été cultivés puis mis en culture dans des plaques à 12 puits (Labtecks). Lorsque les cellules sont arrivées à 80 % de confluence, une solution contenant le peptide de séquence Arg-Gly-Ser à la concentration de 10⁻⁶ M a été appliquée sur les cellules pendant des durées de 5, 15, 30 et 60 minutes. Des tests contrôles ont été effectués en appliquant, sur les cellules, des solutions ne contenant pas d'actif.

Le test a, de la même manière, été effectué sur des fibroblastes 3T3-L1 en culture. Ces cellules ont la particularité de se différencier, sous l'action d'un cocktail hormonal, en préadipocytes puis en adipocytes chargés de triglycérides. Les cellules préadipocytaires sont ensemencées dans des Labteks et entretenues jusqu'à 100 % de confluence en milieu de culture DMEM 10 % et SVF. Arrivées à confluence, les cellules sont alors cultivées en milieu de culture classique, contenant des inducteurs de différenciation (IBMX, dexaméthasone et insuline), de façon à passer en phases de différentiation terminale et à obtenir ainsi des adipocytes matures. Les adipocytes sont alors traités avec le peptide de séquence Arg-Gly-Ser, représentatif de la famille de peptide selon l'invention, mis en solution à 1 % d'une solution à 50 ppm, ou avec une solution ne contenant pas l'actif. Les adipocytes sont incubés pendant des durées différentes : 5 minutes, 15 minutes et 3 heures.

A la fin du temps d'incubation, les différentes plaques, contenant les fibroblastes ou les adipocytes, sont vidées, puis rincées avec 2 mL de PBS froid puis 250 µL d'un tampon de lyse, fournis par le kit, sont ajoutés. Les cellules sont ensuite récoltées, séparément, dans des tubes de 14 mL et chaque puits est rincé avec 2x500 µL de PBS froid. Chaque tube est ensuite passé au polytron durant 10 secondes à 18000 tr/min. Une dilution au 1/1200ème est effectuée pour chaque condition, avec du PBS froid, avant chaque lecture.

Le dosage d'ATP est réalisé sur ces échantillons : 50 µL de cette dilution sont déposés dans une luma-cuvette et 50 µL de luminol sont ajoutés. Après 10 secondes, la lecture de la luminescence est déclenchée. Les mesures sont effectuées à l'aide d'un appareil : le Biocounter M2010A LUMAC®/3M.

Les résultats obtenus expriment le pourcentage d'augmentation de la luminescence. Cette luminescence a été mesurée après différentes périodes d'incubation, dans les cellules traitées avec les actifs par rapport aux cellules non traitées, dans les fibroblastes ainsi que dans les adipocytes. Cette augmentation de luminescence exprime l'augmentation de la quantité d'ATP intracellulaire, la luminescence étant proportionnelle à la quantité d'ATP présente dans les cellules.

| Temps | t = 5 min | t = 15 min | t = 30 min | t = 3 heures |
|---|---|---|---|---|
| Sur fibroblastes traités avec actif | + 60 %, | + 75 % | + 45 % | + 0,2 % |
| Sur adipocytes traités avec actif | + 1 % | + 30 % | - | + 16 % |

Ces résultats démontrent qu'en présence du peptide la quantité d'ATP intracellulaire augmente significativement par rapport aux cellules non traitées avec l'actif. On observe cette hausse de luminescence ainsi bien dans les fibroblastes que dans les adipocytes, ce qui signifie que l'augmentation d'ATP s'effectue aussi bien dans les deux types de cellules.

Cependant, au niveau des fibroblastes l'augmentation débute à 5 minutes et atteint son maximum 15 minutes après l'administration de l'actif selon l'invention, pour revenir approximativement à son état basal au bout de 2 heures. Tandis qu'au niveau des adipocytes, le maximum est atteint 15 minutes après l'application de l'actif et le taux d'ATP se maintient pendant, au moins, 3 heures.

### Exemple 2 - Mise en évidence de l'effet du peptide sur l'expression des protéines de la matrice extracellulaire.

Le but de l'étude est de déterminer l'influence du peptide selon l'invention sur la synthèse de fibronectine, sur la synthèse de collagène de type I et de type III, par les fibroblastes, à l'aide de la technique d'immunofluorescence.

L'immunofluorescence est une technique semi-quantitative qui permet d'apprécier le taux de chacune des protéines présentes dans le cytoplasme cellulaire.

Des fibroblastes humains sont ensemencés dans des Labteks puis mis en culture pendant une nuit. Lorsque les cellules sont arrivées à entre 40 et 70 %, environ, de confluence, après rinçage avec du tampon HBS, une composition contenant une concentration de 10⁻⁶ M du peptide de séquence Arg-Gly-Ser, représentatif de la famille de peptides selon l'invention, ou une composition contrôle ne contenant pas de peptide, est ajoutée. Les cellules sont ensuite incubées durant 24 heures. Après élimination des surnageants et rinçage des cultures, les cellules sont fixées avec du paraformaldhéyde pendant 30 minutes à 4°C puis rincées avec du tampon PBS. 200µL d'anticorps anti-fibronectine et/ou d'anticorps anti-collagène I et/ou d'anticorps anti-collagène III sont alors ajoutés. L'incubation dure 30 minutes à température ambiante. Les surnageants sont éliminés et les cellules sont rincées au PBS. 200µL d'anticorps secondaire, couplé à un marqueur fluorescent (la fluorescéine) est ensuite additionné. Après 30 minutes d'incubation à température ambiante, les surnageants sont éliminés et les cellules sont rincées au PBS. Les lames sont alors montées puis examinées au microscope à fluorescence inversée. Les quantités de fibronectine et/ou de collagène de type I et/ou de type III, synthétisées par les cellules, sont proportionnelles à l'intensité de la fluorescence.

Les résultats obtenus démontrent que l'ajout de peptides dans le milieu de culture des fibroblastes a pour effet d'augmenter la synthèse de fibronectine et/ou la synthèse de collagène de type I et de type III par les cellules. Cette stimulation a été observée de manière importante.

En effet, lorsque les fibroblastes sont incubés en présence de la composition contenant le peptide, on assiste au bout de 24 heures à une augmentation de l'intensité de la fluorescence, par rapport aux cellules non traitées, traduisant ainsi une stimulation de la synthèse de la fibronectine et/ou une stimulation de la synthèse du collagène de type I et/ou une stimulation de la synthèse du collagène de type III par les fibroblastes.

### Exemple 3 - Mise en évidence par immunofluorescence de l'effet du peptide sur l'expression des kératines.

Le but de l'étude est de déterminer l'influence du peptide selon l'invention sur la synthèse de kératines, par les kératinocytes, à l'aide de la technique d'immunofluorescence.

Une étude par la technique d'immunofluorescence, identique à celle de l'exemple 2, a été effectuée sur des kératinocytes humains HaCat, avec des anticorps anti-kératines Pan cK.

Les résultats obtenus démontrent que l'ajout du peptide de séquence Arg-Gly-Ser, représentatif de la famille de peptide selon l'invention, dans le milieu de culture des kératinocytes a pour effet d'augmenter la synthèse de kératine par les cellules. Cette stimulation a été observée de manière importante. En effet, lorsque les kératinocytes sont incubés en présence de la composition contenant les actifs, on assiste, au bout de 24 heures, à une augmentation de l'intensité de la fluorescence traduisant ainsi une stimulation de la synthèse de kératine par les kératinocytes.

### Exemple 4 - Mise en évidence de l'effet du peptide sur la différenciation cellulaire.

Le but de l'étude est de déterminer l'influence du peptide selon l'invention sur la différenciation cellulaire, par la technique d'immunofluorescence. Le principe de cette technique repose sur la détection de marqueurs de la différenciation cellulaire comme, par exemple, l'involucrine et la molécule ERG.

Une étude, par la technique d'immunofluorescence identique à celle des exemples 2 et 3, a été effectuée sur des kératinocytes humains HaCat, avec des anticorps anti-ERG 1-2 (la molécule ERG 1-2 étant marqueur de différenciation cellulaire très précoce).

Les résultats obtenus démontrent que l'ajout du peptide de séquence Arg-Gly-Ser, représentatif de la famille de peptide selon l'invention, dans le milieu de culture des kératinocytes a pour effet d'augmenter la quantité de molécules ERG présente dans les cellules. Cette stimulation a été observée de manière importante. En effet, lorsque les kératinocytes sont incubés en présence de la composition contenant le peptide, on assiste, au bout de 24 heures, à une augmentation de l'intensité de la fluorescence traduisant ainsi une stimulation de la synthèse de molécules ERG par les kératinocytes donc une augmentation due au taux de différenciation cellulaire.

### Exemple 5 : Mise en évidence de l'activité du peptide au niveau des adipocytes.

### 1) Principe du test in-vitro :

L'effet du peptide est évalué par observation microscopique du nombre et de la taille des vacuoles lipidiques présents dans les adipocytes après coloration. Les adipocytes étant incubés, ou non, en présence de la substance à tester.

### 2) Modèle expérimental :

La mise en évidence de l'activité biologique du principe actif a été réalisée sur la lignée cellulaire préadipocytaire 3T3-L1, maintenue dans un milieu adéquat. Ces préadipocytes étant capables de rentrer, dans certaines conditions, en phase de différentiation terminale.

Les cellules sont ensemencées dans des Labteks et entretenues jusqu'à 100 % de confluence en milieu de culture DMEM 10 % et SVF. Arrivées à confluence, les cellules sont alors cultivées en milieu de culture classique, contenant des inducteurs de différenciation (IBMX, dexaméthasone et insuline), de façon à passer en phase de différentiation terminale et à obtenir ainsi des adipocytes matures. Les adipocytes sont alors traités avec le peptide de séquence Arg-Gly-Ser, représentatif de la famille de peptide selon l'invention, mis en solution à 1 % d'une solution à 50 ppm. Les adipocytes sont incubés pendant des durées différentes : 30 minutes, 3 heures et 6 heures. Après les différentes périodes d'incubation, en présence, ou non, de l'actif à tester, les adipocytes sont colorés avec la solution « Oil Red » (*Sigma,* O-0625). Cette solution est préparée par ajout de 0,5g de produit dans 100mL d'isopropanol et par dilution au 4/10 dans de l'eau distillée, puis filtration. Les adipocytes sont fixés durant 10 minutes dans une solution de formol 4 % et NaCl, la solution Red Oil est appliquée durant 15 minutes. Une contre-coloration à l'Hématoxyline, durant 30 secondes, est possible. Les cellules sont ensuite rincées avec de l'eau tiède et montées sur lames, en milieu hydrophile (*Aquatex*). L'observation est effectuée au microscope optique.

### 3) Résultats :

Les résultats de l'observation des cellules démontrent que, contrairement aux adipocytes matures témoins (sur lesquels l'actif n'a pas été appliqué), qui ont une forme volumineuse sphérique et une accumulation importante de vésicules lipidiques intracytoplasmique ; les adipocytes matures, traités par une solution contenant l'actif selon l'invention, ont une morphologie moins arrondie et un contenu en vésicules lipidiques intra-adipocytaires nettement diminué.

La solution contenant le peptide selon l'invention s'avère donc particulièrement efficace dans le processus de limitation de l'hypertrophie adipocytaire en augmentant, vraisemblablement, le phénomène de lipolyse. Il permet donc une élimination des triglycérides contenus dans les vésicules intra-adipocytaires.

Ces résultats sont confirmés par le dosage du taux de glycérol relargué par les adipocytes dans le milieu surnageant, selon la technique d'analyse par HPLC, ainsi que par une technique de dosage enzymatique.

### Exemple 6: Mise en évidence de l'activité du peptide sur la quantité d'AMPc intracellulaire.

### 1) Principe du test :

L'objectif de ce test est de mesurer l'augmentation de la concentration d'AMPc intracellulaire, dans les adipocytes, afin d'en déduire l'activation de phénomène de lipolyse.

La lipolyse est un mécanisme qui libère les triglycérides contenus dans les vacuoles des adipocytes. Cette réaction s'effectue grâce à une enzyme, la Triglycéride-lipase, qui scinde les triglycérides en acides gras libres et en glycérol. Ces derniers sont relargués dans le milieu extracellulaire et éliminés dans la circulation sanguine. La Triglycéride-lipase est régulée par le taux d'AMPc intracellulaire. Ainsi, en augmentant le taux d'AMPc intracellulaire, on augmente l'activité de l'enzyme permettant l'hydrolyse des triglycérides et on stimule ainsi le phénomène de lipolyse.

### 2) Modèle expérimental :

Le test a été effectué sur des fibroblastes 3T3-L1 en culture. Ces cellules ont la particularité de se différencier, sous l'action d'un cocktail hormonal, en pré-adipocytes puis en adipocytes chargés de triglycérides.

Les cellules 3T3-L1 sont ensemencées sur des plaques de 24 puits et entretenues jusqu'à 100 % de confluence en milieu de culture DMEM 10 % et SVF. Arrivées à confluence, les cellules sont alors cultivées en milieu de culture classique, contenant des inducteurs de différenciation (IBMX, dexaméthasone et insuline), de façon à passer en phases de différentiation terminale et à obtenir ainsi des adipocytes matures. Les adipocytes sont alors traités avec le peptide de séquence Arg-Gly-Ser, représentatif de la famille de peptide selon l'invention, mis en solution à 1 % d'une solution à 50 ppm. Un test négatif contrôle est réalisé en présence d'une solution ne contenant pas d'actif. Les adipocytes sont incubés en présence, ou non, de l'actif à tester. La quantité d'AMPc intracellulaire est mesurée à différentes périodes (15 minutes, 30 minutes et 2 heures).

Après les différentes périodes d'incubation, en présence, ou non, de l'actif à tester, la quantité d'AMPc contenue dans les adipocytes est mesurée à l'aide du Kit « cAMP Biotrak® EIA System » de Amersham Biosciences. A la fin du temps d'incubation, les plaques sont vidées, puis rincées avec du PBS froid. Le protocole de dosage de l'AMPc est réalisé suivant les instructions fournis par le kit. Le dosage de l'AMPc est effectué grâce à la lecture de la DO à 450 nm (la quantité d'AMPc étant proportionnelle à la DO mesurée).

### 3) Résultats :

Les résultats expriment la concentration intracellulaire en AMPc, en nmol/mL, relevée après différent temps d'incubation, ainsi que le pourcentage d'augmentation de la concentration dans les cellules traitées avec les actifs par rapport aux cellules non traitées.

| Temps | t = 0 | t = 15 min | t = 30 min | t=2h. |
|---|---|---|---|---|
| Concentration en nmol/mL | 226 | 246 | 304 | 287 |
| % d'augmentation | - | +86% | +33% | +28% |

Ces résultats démontrent qu'en présence de l'actif, la quantité d'AMPc intracellulaire augmente significativement par rapport aux cellules non traitées avec les actifs. Cette augmentation débute à 5 minutes, environ, et atteint son maximum 30 minutes après l'administration du peptide selon l'invention. On n'observe aucune augmentation de la concentration sur les cellules non traitées avec l'actif.

Cette augmentation de la quantité d'AMPc dans les adipocytes, combinée aux résultats obtenus dans l'exemple 5, permet de conclure que le peptide selon l'invention agit, au niveau des adipocytes, sur le mécanisme de la lipolyse. En effet, en augmentant la quantité d'AMPc intracellulaire dans les adipocytes, on augmente l'activité de la Triglycéride-lipase, et on favorise ainsi la lipolyse. Le peptide selon l'invention favorise donc l'élimination des triglycérides contenus dans les vésicules intra-adipocytaires.

### Exemple 7 - Préparation de compositions.

Ces compositions ont été obtenues par simple mélange des différents composants. Les quantités indiquées sont données en pourcentage de poids.

### 1. Emulsion huile-dans-eau

| ***Noms commerciaux*** | ***Noms INCI*** | ***% massique*** |
|---|---|---|
| ***PHASE HUILEUSE*** | | |
| Montanov 68 | Cetearyl Alcohol (and) Cetearyl Glucoside | 5.00 |
| Huile de Jojoba | Simmondsia Chimensis Seed Oil | 5.00 |
| Huile de Vaseline | Paraffinum Liquidum (Mineral oil) | 5.00 |
| Isopropyl Palmitate | Isopropyl Palmitate | 7.00 |
| ***PHASE AQUEUSE*** | | |
| Glycerine | Glycerin | 5.00 |
| Allantoïne | Allantoin | 0.10 |
| peptide Arg-Gly-Ser | | 1,5 ppm |
| Sepigel 305 | Polyacrylamide (and) C13-14 Isoparaffin (and) Laureth-7 | 0,30 |
| Conservateur | | 0,50 |
| Parfum | Parfum (Fragrance) | 0,50 |
| Eau déminéralisée | Aqua (Water) | Qsp |

### 2. Lotion

| ***Noms commerciaux*** | ***Noms INCI*** | ***% massique*** |
|---|---|---|
| Mono Propylene Glycol | Propylene Glycol | 1,00 |
| Allantoïne | Allantoin | 0,30 |
| Glycérine | Glycerin | 1,00 |
| Cetiol HE | PEG-7 Glyceryl Cocoate | 1,00 |
| Peptide Arg-Gly-Ser | | 0,1 ppm |
| Conservateur | | 0,20 |
| Parfum | Parfum (Fragrance) | 0,50 |
| Eau déminéralisée | Aqua (Water) | qsp |

### 3. Gel

| ***Noms commerciaux*** | ***Noms INCI*** | ***% massique*** |
|---|---|---|
| Carbopol Ultrez 10 ( 2% ) | Carbomer | 25.00 |
| Triéthanolamine | triethanolamine | 0,50 |
| Peptide Arg-Gly-Ser | | 1 ppm |
| Conservateur | | 0,20 |
| EDTA | Tetrasodium EDTA | 0.10 |
| Parfum | Parfum (Fragrance) | 0,50 |
| Colorant hydrosoluble | | Qsp |
| Eau déminéralisée | Aqua (Water) | Qsp |

### 4. Crème amincissante

| ***Noms commerciaux*** | ***Noms INCI*** | **% *massique*** |
|---|---|---|
| ***PHASE A*** | | |
| Montanov 68 | Cetearyl Alcohol (and) Cetearyl Glucoside | 5.00 |
| Squalane | Squalane | 2.50 |
| DUB IPP | Isopropyl Palmitate | 3.50 |
| Eutanol G | Octyldodecanol | 1.50 |
| Phenonip | Phenoxyethanol (and) Methylparaben (and) Ethylparaben (and) Butylparaben (and) Propylparaben (and) Isobutylparaben | 0.50 |
| ***PHASE B*** | | |
| Eau déminéralisée | Aqua (Water) | Qsp |
| Glycerine | Glycerin | 3.00 |
| Butylene Glycol | Butylene glycol | 3.00 |
| ***PHASE C*** | | |
| Simulgel EG | Sodium Acrylate/Acryloyldimethyl Taurate Copolymer(and) Isohexadecane(and) Polysorbate 80 | 0.60 |
| ***PHASE D*** | | |
| Peptide Arg-Gly-Ser | | 1.25 ppm |
| Parfum | Parfum (Fragrance) | Qsp |
| Colorant | | Qsp |

Les constituants de la phase A sont fondus à 75°C et les constituants de la phase B chauffés à 75°C. La phase A est émulsionnée à B, puis le mélange est refroidi en dessous de 40°C. Les phases C et D sont ensuite additionnées sous agitation constante.

### 5. Spray Raffermissant - Amincissant

| ***Noms commerciaux*** | ***Noms INCI*** | ***% massique*** |
|---|---|---|
| ***PHASE A*** | | |
| Emulgade SEV | Glyceryl Stearate (and) Ceteareth-20 (and) Ceteareth-12 (and) Cetearyl Alcohol | 4.60 |
| Eumulgin B2 | Ceteareth-20 | 1.40 |
| Cetiol OE | Dicaprylyl Ether | 3.00 |
| DUB B1215 | C12-C15 Alkyl Benzoate | 5.00 |
| Phenonip | Phenoxyethanol (and) Methylparaben (and) Ethylparaben (and) Butylparaben (and) Propylparaben (and) Isobutylparaben | 0.50 |
| DUB ININ | Isononyl Isononanoate | 5.00 |
| Phenonip | Phenoxyethanol (and) Methylparaben (and) Ethylparaben (and) Butylparaben (and) Propylparaben (and) Isobutylparaben | 0.50 |
| ***PHASE B*** | | |
| Eau déminéralisée | Aqua (Water) | 15.00 |
| Glycerine | Glycerin | 3.00 |
| ***PHASE C*** | | |
| Eau déminéralisée | Aqua (Water) | Qsp |
| ***PHASE D*** | | |
| Peptide Arg-Gly-Ser | | 1.50 ppm |
| Parfum | Parfum (Fragrance) | qsp |
| Colorant | | qsp |

Les constituants de la phase A et de la phase B sont chauffés séparément à 65°C ; la phase B est incorporée à la phase A sous agitation. La température du mélange est montée à 83°C puis il est refroidi jusqu'à une température d'inversion de phase. La phase C est ensuite additionnée. L'actif est incorporé lorsque la température atteint moins de 40°C. Il est alors possible d'ajouter des parfums et/ou des colorants.

### 6. Gel Raffermissant - Amincissant - anti-cellulite

| ***Noms commerciaux*** | ***Noms INCI*** | ***% massique*** |
|---|---|---|
| Carbopol Ultrez 10 (2%) | Carbomer | 25.00 |
| Eau déminéralisée | Aqua (Water) | Qsp |
| DUB DIOL | Methyl Propanediol | 3.00 |
| EDTA | Tetrasodium EDTA | 0.10 |
| Glydant Plus Liquid | DMDM Hydantoïn (and) Iodopropynyl butylcarbamate | 0.20 |
| Peptide Arg-Gly-Ser | | 1.25 ppm |
| TEA | Triethanolamine | 0.50 |
| Parfum | Parfum (Fragrance) | Qsp |
| Colorant hydrosoluble | | Qsp |

Le gel de Carbopol est préparé à 2 %. Les ingrédients sont ajoutés dans l'ordre énuméré ci-dessus, sous agitation. Le mélange est ensuite neutralisé avec la TEA. Le parfum et les colorants sont ajoutés si nécessaire.

## Revendications

1. Composition cosmétique et/ou dermatologique et/ou pharmaceutique, **caractérisée en ce qu'**elle contient, dans un milieu acceptable, comme principe actif, au moins un peptide de formule (I):
(AA)n-Arg-Gly-Ser-(AA)n (I)
dans laquelle (AA) est un quelconque acide aminé ou un de ses dérivés, n est un entier compris entre O et 3, le peptide étant présent dans la composition à une concentration comprise entre 0,005 et 500 ppm environ.

2. Composition selon la revendication 1 **caractérisée en ce que** le peptide est la séquence Arg-Gly-Ser.

3. Composition selon l'une quelconque des revendications 1 ou 2, **caractérisée en ce que** le peptide est choisi parmi les peptides dont au moins un groupement fonctionnel est protégé par un groupement protecteur, ce groupement protecteur étant soit une acylation ou une acétylation de l'extrémité amino-terminale, soit sur une amidation ou une estérification de l'extrémité carboxy-terminale, soit les deux.

4. Composition selon l'une quelconque des revendications précédentes, **caractérisée en ce que** le peptide est présent dans la composition à une concentration comprise entre 0,1 et 50 ppm environ par rapport au poids total de la composition finale.

5. Composition selon l'une quelconque des revendications précédentes, **caractérisée en ce qu'**elle se présente sous la forme d'une composition cosmétique et/ou dermatologique adaptée à l'administration par voie topique cutanée comprenant un milieu cosmétiquement ou pharmaceutiquement acceptable.

6. Composition selon l'une quelconque des revendications précédentes, **caractérisée en ce que** le peptide est préalablement solubilisé dans un ou plusieurs solvants cosmétiquement ou pharmaceutiquement acceptables comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants.

7. Composition selon l'une quelconque des revendications précédentes, **caractérisée en ce que** le peptide est préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

8. Composition selon l'une quelconque des revendications précédentes, **caractérisée en ce qu'**elle se présente sous forme d'une solution aqueuse, hydralcoolique ou huileuse ou sous la forme d'une émulsion huile-dans-eau, eau-dans-huile ou émulsions multiples ou sous forme de crème, de suspensions, ou encore poudres; ces compositions pouvant être plus ou moins fluides ou solides et avoir l'aspect d'une crème, d'une lotion, d'un lait, d'un sérum, d'une pommade, d'un gel, d'une pâte, d'une mousse ou d'un stick.

9. Procédé de traitement cosmétique non thérapeutique consistant à appliquer une quantité efficace d'une composition telle que définie selon l'une quelconque des revendications 1 à 8, pour le soin et le traitement de la peau et/ou des phanères permettant d'activer le métabolisme énergétique cellulaire.

10. Procédé de traitement cosmétique non thérapeutique consistant à appliquer une quantité efficace d'une composition telle que définie selon l'une quelconque des revendications 1 à 8, pour le soin et le traitement de la peau et/ou des phanères présentant des propriétés cytostimulantes, notamment en tant qu'agent de soin favorisant la régénération tissulaire.

11. Procédé de traitement cosmétique non thérapeutique consistant à appliquer une quantité efficace d'une composition telle que définie selon l'une quelconque des revendications 1 à 8, pour stimuler la synthèse des protéines de la matrice extracellulaire et/ou stimuler la synthèse de kératines.

12. Procédé de traitement cosmétique non thérapeutique consistant à appliquer une quantité efficace d'une composition telle que définie selon l'une quelconque des revendications 1 à 8, pour lutter et/ou prévenir contre les manifestations du vieillissement cutané.

13. Procédé de traitement cosmétique non thérapeutique consistant à appliquer une quantité efficace d'une composition telle que définie selon l'une quelconque des revendications 1 à 8, pour protéger la peau et/ou les cheveux contre tous types d'agressions extérieures.

14. Procédé de traitement cosmétique non thérapeutique consistant à appliquer une quantité efficace d'une composition telle que définie selon l'une quelconque des revendications 1 à 8, contre la cellulite et/ou la peau d'orange; et/ou afin de réduire, éliminer ou prévenir les surcharges graisseuses sous-cutanées.

## Claims

1. Cosmetic and/or dermatological and/or pharmaceutical composition, **characterised in that**, in an acceptable medium, it contains at least one peptide of formula (I) as an active principle:
(AA) n-Arg-Gly-Ser- (AA) n (I)
wherein (AA) is any amino acid or one of the derivatives thereof, n is an integer between 0 and 3, the peptide being present in the composition at a concentration of between approximately 0.005 and 500 ppm.

2. Composition according to claim 1, **characterised in that** the peptide is the sequence Arg-Gly-Ser.

3. Composition according to any of claims 1 or 2, **characterised in that** the peptide is chosen from amongst the peptides at least one functional group of which is protected by a protective group, said protective group being either an acylation or acetylation of the aminoterminal end, or an amidation or esterification of the carboxy-terminal end, or both.

4. Composition according to any of the preceding claims, **characterised in that** the peptide is present in the composition at a concentration of between approximately 0.1 and 50 ppm in relation to the total weight of the final composition.

5. Composition according to any of the preceding claims, **characterised in that** same is in the form of a cosmetic and/or dermatological composition suitable for topical cutaneous administration and including a cosmetically or pharmaceutically acceptable medium.

6. Composition according any of the preceding claims, **characterised in that** the peptide is pre-dissolved in one or more cosmetically or pharmaceutically acceptable solvents such as water, ethanol, propylene glycol, butylene glycol, dipropylene glycol, ethoxylated or propoxylated diglycols, cyclic polyols, petroleum jelly, a vegetable oil or any mixture of said solvents.

7. Composition according to any of the preceding claims, **characterised in that** the peptide is pre-dissolved in a cosmetic or pharmaceutical carrier such as liposomes or adsorbed by powdered organic polymers, mineral substrates such as talcs and bentonites, or more generally dissolved in, or attached to any cosmetically or pharmaceutically acceptable carrier.

8. Composition according to any of the preceding claims, **characterised in that** same is in the form of an aqueous, hydro-alcoholic or oil solution, or in the form of an oil-in-water, water-in-oil emulsion or multiple emulsions, or in the form of a cream, suspensions, or else powders; said compositions having the capability of being more or less fluid or solid and of having the appearance of a cream, lotion, milk, serum, pomade, gel, paste, foam or stick.

9. Non-therapeutic cosmetic treatment method consisting in applying an effective quantity of a composition as defined according to any of claims 1 to 8, for the care and treatment of the skin and/or appendages of the skin, thereby enabling the cell energy metabolism to be activated.

10. Non-therapeutic cosmetic treatment method consisting in applying an effective quantity of a composition as defined according to any of claims 1 to 8, for the care and treatment of the skin and/or appendages of the skin, having cytostimulating properties, in particular as a care agent promoting tissue regeneration.

11. Non-therapeutic cosmetic treatment method consisting in applying an effective quantity of a composition as defined according to any of claims 1 to 8, for stimulating protein synthesis of the extracellular matrix and/or stimulating keratin synthesis.

12. Non-therapeutic cosmetic treatment method consisting in applying an effective quantity of a composition as defined according to any of claims 1 to 8, for combating and/or preventing the effects of skin ageing.

13. Non-therapeutic cosmetic treatment method consisting in applying an effective quantity of a composition as defined according to any of claims 1 to 8, for protecting the skin and/or hair against any type of external stresses.

14. Non-therapeutic cosmetic treatment method consisting in applying an effective quantity of a composition as defined according to any of claims 1 to 8, against cellulitis or orange skin; and/or in order to reduce, eliminate or prevent subcutaneous fat overload.

## Patentansprüche

1. Kosmetische und / oder dermatologische und / oder pharmazeutische Zusammensetzung, **dadurch gekennzeichnet, dass** sie, in einem akzeptablen Milieu, als Wirkstoff mindestens ein Peptid mit der folgenden Formel (I) enthält:
(AA)n-Arg-Gly-Ser-(AA)n (I)
in der (AA) irgendeine Aminosäure oder eines ihrer Derivate ist, n eine ganze Zahl, enthalten zwischen 0 und 3 ist, wobei das Peptid in der Zusammensetzung in einer Konzentration zwischen ungefähr 0,005 und 500 ppm vorhanden ist.

2. Zusammensetzung nach Anspruch 1, **dadurch gekennzeichnet, dass** das Peptid die Sequenz Arg-Gly-Ser ist.

3. Zusammensetzung nach einem der Ansprüche 1 oder 2, **dadurch gekennzeichnet, dass** das Peptid ausgewählt ist aus den Peptiden, bei denen mindestens eine funktionelle Gruppe von einer Schutzgruppe geschützt wird, wobei diese Schutzgruppe entweder eine Acylierung oder eine Acetylierung des aminoterminalen Endes ist oder auf einer Amidbildung oder Veresterung des carboxyterminalen Endes, oder beides ist.

4. Zusammensetzung nach einem der vorhergehenden Ansprüche, **dadurch gekennzeichnet, dass** das Peptid in der Zusammensetzung in einer Konzentration zwischen ungefähr 0,1 und 50 ppm hinsichtlich des Gesamtgewichts des Endzusammensetzung vorhanden ist.

5. Zusammensetzung nach einem der vorhergehenden Ansprüchen, **dadurch gekennzeichnet, dass** sie die Form einer kosmetischen und / oder dermatologischen Zusammensetzung aufweist, die angepasst ist, um auf topischem kutanem Weg verabreicht zu werden, umfassend ein kosmetisch oder pharmazeutisch akzeptables Milieu.

6. Zusammensetzung nach einem der vorhergehenden Ansprüche, **dadurch gekennzeichnet, dass** das Peptid zuvor in einem oder in mehreren kosmetisch oder pharmazeutisch akzeptablen Lösemitteln wie z.B. Wasser, Ethanol, Propylenglykol, Butylenglykol, Dipropylenglykol, ethoxylierten oder propoxylierten Diglykolen, zyklischen Polyolen, Vaselin, einem pflanzlichen Öl oder jeder Mischung dieser Lösemittel solubilisiert wird.

7. Zusammensetzung nach einem der vorhergehenden Ansprüche, **dadurch gekennzeichnet, dass** das Peptid vorher in einem kosmetischen oder pharmazeutischen Vektor wie z.B. Liposomen solubilisiert oder auf pulvrigen organischen Polymeren, mineralischen Trägern wie z.B. Talk und Bentonit, adsorbiert wird und allgemeiner in jedem kosmetisch oder pharmazeutisch akzeptablen Vektor solubilisiert oder darauf festgesetzt wird.

8. Zusammensetzung nach einem der vorhergehenden Ansprüche, **dadurch gekennzeichnet, dass** sie in Form einer wässrigen, hydralkoholischen oder öligen Lösung oder in Form einer Öl-in-Wasser-, Wasser-in-Öl Emulsion oder von Mehrfachemulsionen oder in Form einer Creme, von Suspensionen oder auch Pulver vorliegt; diese Zusammensetzungen können mehr oder weniger flüssig oder fest sein und das Aussehen einer Creme, einer Lotion, einer Milch, eines Serums, einer Pomade, eines Gels, einer Paste, eines Schaums oder eines Sticks aufweisen.

9. Verfahren zur kosmetischen nicht therapeutischen Behandlung zur Anwendung einer wirksamen Menge einer Zusammensetzung, wie gemäß einem der Ansprüche 1 bis 8 definiert, zur Pflege und Behandlung der Haut und / oder der Hautanhangsgebilde, die es ermöglicht, den zellulären Energiestoffwechsel zu aktivieren.

10. Verfahren zur kosmetischen nicht therapeutischen Behandlung zur Anwendung einer wirksamen Menge einer Zusammensetzung, wie gemäß einem der Ansprüche 1 bis 8 definiert, zur Pflege und Behandlung der Haut und / oder der Hautanhangsgebilde, die zytostimulierende Eigenschaften aufweist, insbesondere als Pflegemittel, das die Gewebeerneuerung fördert.

11. Verfahren zur kosmetischen nicht therapeutischen Behandlung zur Anwendung einer wirksamen Menge einer Zusammensetzung, wie gemäß einem der Ansprüche 1 bis 8 definiert, um die Synthese der Proteine der extrazellulären Matrix zu stimulieren und / oder die Synthese von Keratinen zu stimulieren.

12. Verfahren zur kosmetischen nicht therapeutischen Behandlung zur Anwendung einer wirksamen Menge einer Zusammensetzung, wie gemäß einem der Ansprüche 1 bis 8 definiert, um gegen die Erscheinungen des Alterns der Haut zu kämpfen und / oder diesen vorzubeugen.

13. Verfahren zur kosmetischen nicht therapeutischen Behandlung zur Anwendung einer wirksamen Menge einer Zusammensetzung, wie gemäß einem der Ansprüche 1 bis 8 definiert, um die Haut und / oder die Haare gegen alle Arten von äußeren Aggressionen zu schützen.

14. Verfahren zur kosmetischen nicht therapeutischen Behandlung zur Anwendung einer wirksamen Menge einer Zusammensetzung, wie gemäß einem der Ansprüche 1 bis 8 definiert, gegen Cellulitis und / oder Orangenhaut; und / oder um die subkutanen Fettüberladungen zu beseitigen oder diesen vorzubugen.
